Europäisches Patentamt

European Patent Office

Office européen des brevets

(11) Veröffentlichungsnummer: **0 158 284**
**A2**

# (12) EUROPÄISCHE PATENTANMELDUNG

(21) Anmeldenummer: **85104101.2**

(22) Anmeldetag: **04.04.85**

(51) Int. Cl.⁴: **A 61 K 39/002**
**G 01 N 33/569**

(30) Priorität: **13.04.84 DE 3413978**

(43) Veröffentlichungstag der Anmeldung:
**16.10.85 Patentblatt 85/42**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Hermentin, Peter, Dr.**
**Barfüssertor 30**
**D-3350 Marburg 1(DE)**

(72) Erfinder: **Zwisler, Oswald, Dr.**
**Am Gutshof 6**
**D-3350 Marburg 1(DE)**

(72) Erfinder: **Enders, Burkhard, Dr.**
**Oberer Eichweg 12**
**D-3550 Marburg 1(DE)**

(74) Vertreter: **Beck, Bernhard et al,**
**HOECHST AKTIENGESELLSCHAFT Zentrale**
**Patentabteilung Postfach 80 03 20**
**D-6230 Frankfurt/Main 80(DE)**

(54) **Verfahren zur Gewinnung von Parasiten-Antigenen.**

(57) Es wird ein Verfahren zur Gewinnung von Parasiten-Antigenen aus einem Parasiten-Lysat mittels Adsorption an stabilisierte Erythrozyten beschrieben.

Auf diese Weise erhaltene Antigene können als Impfstoffe oder Diagnostika verwendet werden.

EP 0 158 284 A2

Croydon Printing Company Ltd

BEHRINGWERKE AKTIENGESELLSCHAFT

84/B 008
Dr. Ha/Sd.

0158284

## Verfahren zur Gewinnung von Parasiten-Antigenen

Die Erfindung betrifft ein Verfahren zur adsorptiven Isolierung von Parasiten-Antigenen sowie die Verwendung von stabilisierten Erythrozyten in einem solchen Verfahren. Diese Antigene können zur Herstellung eines Diagnostikums oder Impfstoffs verwendet werden.

Die Entwicklung eines humanmedizinisch anwendbaren Impfstoffs gegen Parasiten stellt bis heute ein unbewältigtes Problem dar. Daher besitzt beispielsweise die Entwicklung eines Impfstoffs gegen die Malaria, insbesondere gegen Malaria tropica (Erreger: Plasmodium falciparum), aber auch gegen Malaria tertiana (Erreger: Plasmodium vivax) für die Humanmedizin hohe Priorität. Aber auch Impfstoffe gegen andere human-pathogene Malaria-Erreger, P. ovale und P. malariae, sowie die Erreger parasitärer Erkrankungen von Tieren, etwa der Babesiose oder Theileriose der Rinder wären von hoher Bedeutung. Es bestand daher die Aufgabe, einen Impfstoff gegen Malaria zu entwickeln.

Es ist bekannt, daß die krankheitsvermittelnden Malaria-Erreger rote Blutzellen befallen, in diesen heranwachsen und sich vermehren, schließlich durch Ruptur der Wirtszellen ins Blut gelangen und dort wieder neue Erythrozyten befallen. Ähnlich spielt sich der Infektionsmodus auch bei den Malaria-Erkrankungen von Tieren sowie bei Piroplasmosen, wie etwa der Babesiose oder Theileriose ab.

Die Bindung invasiver Parasiten, sogenannter Merozoiten, an die Erythrozyten erfolgt über Rezeptoren der Erythrozyt-Oberfläche, bei P. falciparum durch Glycophorin A
und B oder bei P. vivax durch Duffy-Antigene.

Überraschenderweise wurde nun gefunden, daß sich stabilisierte Humanerythrozyten zur Isolierung von Antigenen der Malaria-Erreger P. falciparum und P. vivax eignen.

Darüberhinaus wurde überraschenderweise gefunden, daß stabilisierte Rhesusaffen-Erythrozyten zur Isolierung von Antigenen von Erregern der Malaria-Erreger P. knowlesi oder P. fieldii, sowie stabilisierte Rinder-Erythrozyten zur Isolierung von Antigenen von Erregern der Babesiose, insbesondere Babesia bovis oder B. microti verwendet werden können.

Stabilisierte Erythrozyten können demnach zur Gewinnung von Komponenten von Parasiten verwendet werden, welche sich zur Herstellung von Vaccinen oder Diagnostika eignen.

Für die Isolierung von Antigenen bestimmter Parasiten eignen sich diejenigen stabilisierten Erythozyten am besten, die dem Parasiten bei einer natürlichen Infektion als Wirtszelle dienen.

Es wurde jedoch gefunden, daß sich für die Isolierung von Antigenen bestimmter Parasiten bisweilen auch stabilisierte Erythrozyten anderer Spezies eignen, etwa stabilisierte Humanerythrozyten für die Isolierung von P. knowlesi- oder Babesia bovis-Antigenen, wobei in solchen Fällen kreuzreagierende Antigene isoliert werden können.

Gegenstand der Erfindung ist ein Verfahren zur Gewinnung von Parasiten-Antigenen aus einem Parasiten-Lysat mittels Adsorption, dadurch gekennzeichnet, daß man das Lysat mit stabilisierten Erythrozyten in Kontakt bringt, die Flüssigkeit abtrennt und das Antigen von den Erythrozyten eluiert.

Geeignete stabilisierte Erythrozyten sind Humanerythrozyten oder Erythrozyten tierischen Ursprungs, wie zum Beispiel Affen-, Rinder-, Pferde-, Hunde- oder Mäuse-Erythrozyten, bevorzugt jedoch Humanerythrozyten oder Affen- oder Rindererythrozyten, und besonders bevorzugt Humanerythrozyten.

Geeignete Parasiten sind solche, deren Wirtszellen Erythrozyten sind. Solche Parasiten sind insbesondere Protozoen der Gattung Plasmodium, insbesondere Erreger der Malaria, bevorzugt Erreger der Malaria des Menschen, weiterhin bevorzugt Erreger der Malaria tropica (Erreger: P. falciparum) oder der Malaria tertiana (Erreger: P. vivax), besonders bevorzugt Plasmodium falciparum.

Geeignete Erreger der Malaria sind aber auch Erreger der Affen-Malaria, bevorzugt P. fieldii und P. knowlesi oder auch Erreger der Nager- oder der Vogelmalaria.

In Frage kommende Parasiten sind aber auch Erreger von Piroplasmosen, bevorzugt Erreger der Babesiose und der Theileriose, besonders bevorzugt Babesia bovis und B. microti.

Stabilisierte Erythrozyten sind rote Blutzellen, welche durch Behandlung mit einem chemischen Reagenz gegen lytische Vorgänge resistent gemacht wurden. Solche Reagenzien sind beispielsweise Aldehyde, wie zum Beispiel Glutaraldehyd, Formaldehyd, oder Propanal-2-on, aber auch andere chemische Reagenzien, wie zum Beispiel Chrom-IIIchlorid, Sulfosalizylsäure oder Cyanurchlorid. Ein geeignetes Verfahren ist beispielsweise von Avrameas et al.

(1969), Immunochemistry 6, 67 beschrieben.

Im folgenden wird das Verfahren zur Gewinnung von Parasiten-Antigenen näher beschrieben.

In einem Batch-Verfahren werden in an sich bekannter Weise stabilisierte Erythrozyten (Avrameas et al. siehe oben) mit einem Parasiten-Lysat, welches zum Beispiel durch Extraktion von isolierten Parasiten (Mrema et al. (1982), Exp.Parasitol. 54, 285) oder von parasitierten Erythrozyten, sogenannten Schizonten (Jungery et al. (1983), Nature 301, 704) mit einem geeigneten Puffer-System, wie zum Beispiel physiologische Kochsalzlösung, phosphatgepufferte Kochsalzlösung oder Tris-HCl, pH 7,2, welches einen Protease-Inhibitor wie Aprotinin, TLCK, TPCK, PMSF, PCMB, Chymostatin oder Leupeptin enthalten kann, und welchem ein Detergenz wie Triton®-X 100, Nonidet® P40, Ammonyx® LO, Natriumdesoxycholat, Octylglucosid oder Sulfobetain in einer Konzentration von 0,5 - 10 g/l zugesetzt ist, hergestellt und von korpuskulären Bestandteilen, zum Beispiel durch Zentrifugation bei 100 - 100.000 g, befreit wird, bei einer Temperatur von 0-45°C für einen von der Inkubationstemperatur abhängigen Zeitraum, zum Beispiel 60 min bei 37°C oder 120 min bis mehrere Stunden bei 4°C, inkubiert.

Das Parasiten-Lysat kann aber auch ohne Zusatz eines Detergenz hergestellt werden, indem die Parasiten beispielsweise mechanisch, zum Beispiel durch Ultraschall-Behandlung, Stickstoff-Dekompression, Gefrier-Brechung, Einfrier- und Auftau-Vorgänge oder durch Druck-Anwendung, lysiert werden (Kreier et al. (1977), Bull. WHO 55, 317).

Das Parasiten-Lysat kann aber auch mit einer Kombination beider Extraktions- bzw. Lysis-Verfahren, also durch
mechanische Ruptur in Gegenwart eines Detergenz, hergestellt werden.

Ein verwendbares Lysat kann aber auch durch differentielle Zentrifugation von Kulturüberständen platzender
Schizonten, zum Beispiel 5 Minuten mit 400 g, 15 Minuten
mit 3.500 g und 30 Minuten mit 20.000 g, und nachfolgende oben beschriebene Lysis gewonnen werden.

Ein verwendbares Lysat kann aber auch nach hypotoner
Lysis von Schizonten, welche zum Beispiel in Anlehnung
an die hypotone Lysis von Erythrozyten in bekannter Weise (Dodge et al. (1969), Arch.Biochem.Biophys. 100, 130)
erfolgen kann, gewonnen werden.

Die mit einem Lysat inkubierten Erythrozyten können, gegebenenfalls mehrmals, mit einem oben angegebenen Lysis-
Puffer von ungebundenen Bestandteilen freigewaschen und
nachfolgend in bekannter Weise, etwa durch eine Salzlösung von hoher Konzentration, wie eine 1 mol/l Kochsalzlösung, oder durch chaotrope Gradienten, etwa durch Zusatz von Kaliumrhodanid oder Harnstoff, oder durch Zusatz von Zuckern wie N-acetyl-D-glucosamin oder Methyl-
2-acetamido-2-desoxy-D-glucopyranosid oder durch Erniedrigung des pH-Wertes unter 7, etwa mittels eines Citrat-Puffers pH 5, Glycin-Puffers pH 3 oder Tris-HCl pH
3, oder durch Erhöhung des pH-Wertes über 7, etwa mittels Tris-Puffer pH 8 oder 11 oder einer 10 g/l Ammoniumhydroxidlösung in phosphatgepufferter Kochsalzlösung
(PBS), desorbiert, wobei bei der Eluation Inkubationszeiten von zum Beispiel 1-30 Minuten und Temperaturen von
0-40°C, bevorzugt Raumtemperatur oder 4°C, besonders bevorzugt jedoch 4°C, verwendet werden.

Die Eluate werden gegebenenfalls neutralisiert und/oder dialysiert, die eluierten Proteine charakterisiert, etwa mittels Elektrophorese oder Komplex- oder Immunkomplex-Bildung, gegebenenfalls durch biochemische oder immunologische Methoden, wie zum Beispiel Gelfiltrations-, Ionenaustausch- oder Affinitätschromatographie, gereinigt, gegebenenfalls dialysiert, lyophilisiert oder kristallisiert.

Es wurde überraschenderweise gefunden, daß solche mittels Adsorption an stabilisierten Erythrozyten isolierten Parasitenantigene zur Herstellung von Impfstoffen gegen die jeweilige parasitäre Erkrankung brauchbar sind.

Gegenstand der Erfindung ist deshalb weiterhin die Verwendung eines auf die beschriebene Weise gewonnenen Parasitenantigens zur Herstellung eines Impfstoffs gegen solche Parasiten.

Zur Herstellung eines solchen Impfstoffs gegen Parasiten kann das auf die beschriebene Weise gewonnene Antigen-Gemisch in bekannter Weise, etwa wie vorstehend beschrieben, weiter gereinigt und, je nach parasitärer Spezifität, zu einem Human- oder Veterinärimpfstoff zur aktiven Immunisierung aufgearbeitet werden.

Das auf die beschriebene Weise gewonnene Antigen-Gemisch kann auch zu einem Diagnostikum aufgearbeitet werden.

Die Erfindung wird durch die folgenden Beispiel erläutert:

## Beispiel 1

Herstellung stabiliserter Humanerythrozyten

Humanerythrozyten wurden fünfmal in phosphatgepuffertem Kochsalz pH 7,2 zentrifugiert, und das Sediment mit phosphatgepuffertem Kochsalz auf das zehnfache Volumen gebracht. Dann wurde eine wäßrige Lösung von 250 mg Glutaraldehyd je ml in einer Gesamtmenge von einem Zehntel der Erythrozytensuspension im Eisbad zugetropft, 60 min. im Eisbad und weitere 60 min. bei Raumtemperatur gerührt, die Zellen abzentrifugiert, fünfmal in phosphatgepuffertem Kochsalz pH 7,2 gewaschen, das Zellsediment mit phosphatgepuffertem Kochsalz auf das fünffache Volumen suspendiert und bei fünf Grad Celsius aufbewahrt.
Nach dem selben Verfahren wurden Rinder-, Pferde-, Affenoder Hunde-Erythrozyten stabilisiert.

## Beispiel 2

10 ml eines auf bekannte Weise hergestellten P. falciparum-Merozoiten-Lysats (Mrema et al. (1982), Exp.Parasitol. 54, 285) wurde mit 4 ml stabilisierten Humanerythrozyten vermischt und unter leichter Bewegung 1 Stunde bei 37°C inkubiert. Danach wurden die Erythrozyten bei 37°C mehrmals in dem für die Herstellung des Lysats verwendeten Puffer-System gewaschen und danach bei 4°C mit 0,3 mol/l N-Acetyl-D-glucosamin in 1 mol/l Kochsalzlösung mehrmals extrahiert. Die Extrakte wurden gegen phosphatgepufferte Kochsalzlösung dialysiert, mittels Ultrafiltration konzentriert, mittels Gelfiltration gereinigt und in flüssigem Stickstoff präserviert.
In anderen Ansätzen wurde mindestens 2 Stunden bei 4°C inkubiert und in analoger Weise eluiert. Alternativ wurde auch mit Glycin-HCl, pH 3 eluiert und das Eluat neutralisiert.

Beispiel 3

Eine Suspension von Malaria-Schizonten des Erregers P.
falciparum wurde aus synchronisierter in vitro-Kultur in
bekannter Weise (Jensen (1978), Am.J.Trop.Med.Hyg. 27,
1274) konzentriert, die Schizonten unter physiologischen
Bedingungen mehrmals gewaschen, in bekannter Weise (Jungery et al. (1983), Nature 301, 704) lysiert und die
Malaria-Antigene wie in Beispiel 2 mittels stabilisierten Humanerythrozyten gewonnen und gegebenenfalls weiter
gereinigt.

Beispiel 4

In bekannter Weise isoliert P. falciparum-Schizonten
(Beispiel 3) wurden zur Parasiten- (Merozoiten-)Freisetzung rekultiviert, die freigesetzten Merozoiten in
Abständen von 30 Minuten bis 2 Stunden durch differentielle Zentrifugation bei 350 und 3.000 g pelletiert
und die Malaria-Antigene wie im Beispiel 2 beschrieben
isoliert und gegebenenfalls weiter gereinigt.

Beispiel 5

Plasmodium knowlesi-Merozoiten wurden in bekannter Weise
(Dennis et al. (1975), Parasitology 71, 475) isoliert,
die Antigene in Analogie zu Beispiel 2 unter Verwendung
von stabilisierten Rhesusaffen-Erythrozyten isoliert und
gegebenenfalls weiter gereinigt.

Beispiel 6

Babesia bovis-Merozoiten wurden in Analogie zu Beispiel 2 lysiert und die Antigene unter Verwendung von stabilisierten Rindererythrozyten in Analogie zu Beispiel 2
isoliert.

Beispiel 7

Es wurde entsprechend Beispiel 6 gearbeitet, wobei statt
stabilisierter Rindererythrozyten stabilisierte Humanerythrozyten verwendet wurden.

Patentansprüche:

1. Verfahren zur Gewinnung von Parasiten-Antigenen aus einem Parasiten-Lysat mittels Adsorption, dadurch gekennzeichnet, daß man das Lysat mit stabiliserten Erythrozyten in Kontakt bringt, die Flüssigkeit abtrennt und das Antigen von den Erythrozyten eluiert.

2. Verfahren nach Anspruch 1, dadurch gekennzeichnet, daß die Erythrozyten Humanerythrozyten oder Affen- oder Rindererythrozyten sind.

3. Verfahren nach Anspruch 1, dadurch gekennzeichnet, daß die Parasiten Erreger der Malaria, Babesiose oder der Theileriose sind.

4. Verfahren nach Anspruch 1, dadurch gekennzeichnet, daß die Erythrozyten mit einem Aldehyd stabilisiert sind.

5. Verwendung eines Parasiten-Antigens nach Anspruch 1 zur Herstellung eines Impfstoffs gegen einen solchen Parasiten oder als Diagnostikum.